# EUROPEAN PATENT SPECIFICATION

(11) **EP 0 852 933 B1**
(45) Date of publication and mention of the grant of the patent: **29.03.2000**
(21) Application number: 97304915.8
(22) Date of filing: 04.07.1997
(51) Int. Cl.: A61F 2/06

(54) **Balloon-expandable, crush-resistant locking stent**
Ballonexpandierbarer, druckbeständiger Stent mit Verschluss
Stent avec verrouillage, expansible à ballonnet et résistant à la compression

(30) Priority: 13.01.1997 US 783583
(43) Date of publication of application: 15.07.1998
(73) Proprietor: ADVANCED CARDIOVASCULAR SYSTEMS, INC., Santa Clara California 95052 (US)
(72) Inventor: Cox, Daniel L., Palo Alto, California 94301 (US)
(74) Representative: McLeish, Nicholas Alistair Maxwell

(56) References cited:
- EP-A- 0 423 916
- WO-A-96/41592
- US-A- 4 994 071

## Description

### BACKGROUND OF THE INVENTION

The present invention generally relates to expandable, intraluminal vascular grafts, commonly referred to as stents. More particularly, the present invention relates to a balloon-expandable, crush-resistant locking stent.

Expandable intraluminal vascular grafts, generally called stents, are adapted to be implanted into a body lumen of a patient, such as a blood vessel, to maintain the patency of the vessel. These devices frequently are used in the treatment of atherosclerotic stenosis in blood vessels, especially after percutaneous transluminal coronary angioplasty (PTCA) procedures have been performed, in order to reduce the likelihood of restenosis of a blood vessel.

For expandable stents that are delivered with expandable catheters, such as balloon catheters, the stents are positioned over the balloon portion of the catheter and are expanded from a reduced diameter to an enlarged diameter which is greater than or equal to the diameter of the target artery, by inflating the balloon. Stents of this type can be expanded and held in the enlarged diameter configuration by deformation as is taught in, for example, U.S. Patent No. 4,733,665 to Palmaz; by engagement of the stent walls with respect to each other as, for example, is disclosed in U.S. Patent No. 4,740,207 to Kreamer, U.S. Patent No. 4,877,030 to Beck et al., and U.S. Patent No. 5,007,926 to Derbyshire; and by one-way engagement of the stent walls together with endothelial growth into the stent as is shown in U.S. Patent No. 5,059,211 to Stack et al.

In order to be easily expandable, a number of conventional stents have a rolled-up cylinder construction. For example, U.S. Patent No. 5,443,500 to Sigwart discloses an intravascular stent, intended for implantation in a stenotic area or zone of obstruction of a blood vessel, which consists of a flat sheet that is perforated to form a reticulated or lattice-type structure with undeformable links and which is formed from a malleable material. The sheet is rolled up temporarily and locked into a spiral having a relatively small diameter on a deflated balloon which is mounted on the end of a catheter. The stent is retained in the rolled-up state by a tie laced into overlapping links. Once the stent has been positioned at the restricted area of the blood vessel which is to be treated, and after the tie is removed, the rolled sheet is expanded to a desired diameter by inflating the balloon.

U.S. Patent No. 5,423,885 to Williams discloses an expandable, balloon catheter-delivered intravascular stent having a plurality of protrusions on the outer surface of the stent for engaging the artery walls at the site the stent is deployed. The stent has a rolled-up sheet construction, wherein apertures are formed in the stent body of the stent from the space vacated in the body of the stent by the material forming the protrusions. When the stent is expanded by the balloon catheter, the protrusions engage both the apertures to lock the stent into the expanded diameter and the artery walls to secure it in position.

U.S. Patent No. 5,306,286 to Stack et al. discloses an expandable stent having a rolled-up mesh construction. The stent can be reduced in diameter by a rolling motion while still having a cylindrical configuration on its outer surface for uniform engagement with a vessel wall. The rolled-up, bioabsorbable stent is mounted either on a balloon catheter, a mechanically-expandable catheter, or another suitable stent delivery assembly. By expanding the distal balloon of the catheter or the mechanically-expandable distal end portion of the catheter, the stent is expanded so as to engage the vessel wall.

U.S. Patent No. 5,192,307 to Wall discloses a stent-like prosthesis which is formed of plastic or sheet metal and which is expandable or contractible for placement. The stent selectively may be biased towards a closed position and lockable in an open position or it may be biased in an open position and lockable in a closed position. In the former case, the stent is put into place in its collapsed condition, then forcibly expanded by a balloon to the desired locked condition. In the latter case, the stent may be held by a pin or the like in its collapsed condition, and the pin removed to allow the stent to assume its open position. The locking function is performed by one or more hooks formed into the wall of the device which engage complementary recesses formed in an opposing wall to mechanically interlock the rolled-up sheet forming the stent.

U.S. Patent 5,441,515 to Khosravi et al. discloses an intravascular stent comprising a cylindrical sheet having overlapping edges that interlock. The edges have a series of protrusions and apertures that interlock and ratchet as the stent expands to an open position to support a section of an arterial wall. The stent may be expanded by a balloon catheter or it may be self-expanding. A plurality of retaining members keep the stent open, and a buckle-fastening member is used in one embodiment.

U.S. Patent No. 5,618,299 issued on April 8, 1997 corresponding to EP-A-0,621,017 to Khosravi et al and U.S. Patent No. 5,441,515 to Khosravi et al relate to a ratcheting stent having a rolled, cylindrical sheet construction.

There is, however, still a need for an expandable, crush-resistant locking stent that has a durable, crush-proof construction with a simplified locking mechanism. The crush-proof aspect of the stent minimizes the possibility of an embolism in the vessel if, after implantation, the stent collapses as a result of, for example, an accidental chest trauma or impact on a vessel close to the skin such as the carotid arteries.

### SUMMARY OF THE INVENTION

Embodiments of the present invention provide a method and apparatus directed to an intraluminal, rolled-up crush-resistant stent for implantation in a body lumen comprising a plurality of resilient, substantially parallel struts having a zig-zag configuration and extending from a base section. Each zig-zag strut defines a large amplitude when the strut is in an unstretched state and a small amplitude when the strut is in a stretched state. A plurality of openings are formed within the base section corresponding to each of the zig-zag struts, each opening having a width that is less than the large amplitude. The plurality of struts are curved and, when stretched to the small amplitude, pass through the openings the base section forming a cylindrical hoop.

The crush-resistant stent includes a zig-zag pattern which can have a variety of cross-sections including round, square, rectangular, elliptical, or triangular shapes. Each zig-zag strut extends from a locking fixture to which it is either attached or from which it is made from the same block of material. The ends of each zig-zag strut can be terminated in a variety of configurations. Each zig-zag strut fits through a corresponding slot or opening in the base section or similar locking fixture to complete the cylindrical hoop.

A stent embodying the present invention may be loaded onto a delivery catheter or the like. In its continuous-hoop form, the stent receives the deflated balloon of a balloon catheter. In its incomplete hoop form the zig-zag struts can be wound onto the balloon catheter, and by pulling the zig-zag struts through the respective slots and then releasing the tension, the stent is locked onto the balloon catheter.

The ends of the zig-zag struts may be wound continuously around the balloon and can be held in place with an optional protective sheath. With the protective sheath in place, the balloon catheter and stent, in reduced diameter form, can be transported through the vasculature.

Once positioned at the stenotic region, the stent is deployed first by exposing the stent by withdrawing the protective sheath, and then by inflating the underlying balloon. The zig-zag struts undergo circumferential tensioning from the expanding balloon and assume the smaller amplitude. The smaller amplitude permits the zig-zags to pass through the slots in the base section, thus allowing the hoop diameter to increase following the increasing diameter of the inflating balloon. Further inflation of the balloon and stent implants the stent within the body lumen. After implantation, deflating the balloon facilitates withdrawal of the catheter and completes the stent delivery process.

The stent locks with the base section because the amplitude of each zig-zag strut, being larger than the respective slot width when the zig-zag strut is in the unstressed or untensioned state, abuts against the slot. Conversely, when the stent is being deployed, each zig-zag strut is stretched circumferentially and the amplitude of each zig-zag decreases accordingly until each fits through the width of the slot. At that point, each zig-zag strut passes unobstructed through the slot and the stent undergoes an increase in its diameter.

When the stent is squeezed under a compression load, the stent locks in place because the squeezing action increases the amplitude of each zig-zag to a large amplitude that is too large to fit through its corresponding slot in the base section. Hence, the diameter of the stent thereafter cannot be decreased. If the stent is made from a resilient material, the material in combination with the zig-zag struts abutting the smaller width slots, provide a crush-resistant characteristic to the stent.

In addition, particular forms of the stent can be made to be crush-resistant by using a shape-memory, nickel-titanium alloy as the base material. The stent could be rendered easier to use by the clinician by stabilizing the shape memory in the state at which it is wound onto the balloon. Importantly, a nickel-titanium stent embodying the present invention would be particularly well suited for carotid arteries (or other vessels close to the skin surface) where crush resistance is very important due to possible accidental trauma to the neck area of the patient.

These and other aspects and advantages of the present invention will become apparent from the following detailed description and the accompanying drawings, which illustrate by way of example the features of the invention.

### BRIEF DESCRIPTION OF THE DRAWINGS

FIGURE 1 is a top plan view of a preferred embodiment of the invention illustrating the plurality of coplanar zig-zag struts extending from a base section.

FIG. 2 is an end view of an exemplary embodiment of the base section shown in FIG. 1.

FIG. 3 is an end view of a preferred embodiment after the plurality of zig-zag struts have been bent into a curve and have been inserted through the corresponding slots in the base section to form a hoop.

FIG. 4 is a plan view of an alternative embodiment of the ends of the zig-zag struts.

FIG. 5 is a plan view of the base section shown in FIG. 1.

FIG. 6 is an end view of the base section shown in FIG. 5, in which the slots and the point of attachment of the zig-zag struts to the base section are exposed.

FIGS. 7(a) and (b) are sectional views of two zig-zag struts passing through corresponding slots in the base section wherein FIG. 7(a) shows the struts under circumferential tension, and FIG. 7(b) shows the struts under compression.

### DETAILED DESCRIPTION OF THE PREFERRED EMBODIMENTS

Embodiments of the present invention are directed to an intraluminal, rolled-up crush resistant stent for implantation in a body lumen and a method for loading a crush-resistant stent onto the balloon portion of a catheter. While the present invention is described in detail as applied to intravascular stents, those skilled in the art will appreciate that the present invention can be applied to other surgical procedures and to other internal organs. Moreover, the stent may be deployed in a body lumen through a variety of devices, including but not limited to balloon catheters and specialized stent delivery catheters.

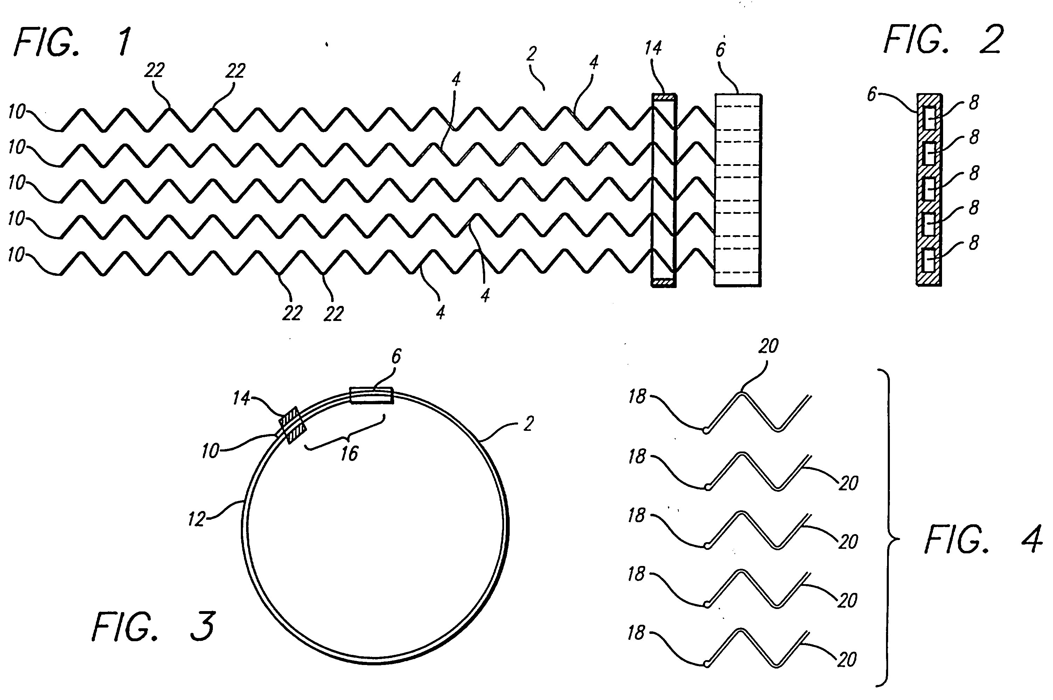

FIG. 1 depicts a plan view of a preferred embodiment of a stent 2 according to the invention. The stent 2 has a plurality of parallel zig-zag struts 4 that also preferably are coplanar. Each zig-zag strut 4 extends from a base section 6. The zig-zag struts 4 are formed as a part of a base section 6 or, alternatively, the struts can be bonded, welded, or mechanically attached to the base section through processes known in the art. As the name implies, each zig-zag strut 4 is wavy along its length wherein the waviness is comprised of a number of individual bends 22. The zig-zag struts 4 preferably are resilient and the material from which each is formed should have some degree of slight shape-memory as well. Further, the zig-zag struts 4 can be characterized by a variety of cross-sections including round (cylindrical), square, elliptical, or triangular shapes. Preferably, the zig-zag struts 4 should have a rectangular cross-section to facilitate bending.

The plan view of FIG. 1 shows the base section 6 with a rectangular shape, although other shapes and dimensions are possible based on design requirements. As is best illustrated in FIG. 2, which is an end view of the base section 6, there are a plurality of slots 8 that correspond to each zig-zag strut 4.

At the terminus of each zig-zag strut 4 is an end 10. Beginning with the coplanar ends 10, the entire plane defined by the zig-zag struts 4 can be curled collectively to form a cylindrical hoop 12, as is best illustrated in the end view of FIG. 3. To close or complete the hoop 12, the ends 10 must pass through the corresponding slots 8 in the base section 6. An optional retainer 14, having a generally rectangular loop shape, permits passage of the zig-zag struts 4 therethrough; after the hoop shape is achieved, an overlapping portion 16 also can pass therethrough and be held in place.

The overlapping portion 16 thus is held against the outside diameter of the hoop 12 as is illustrated in FIG. 3. This prevents ends 10 from extending radially away from the hoop 12, due to the resilience in the material of the zig-zag struts 4.

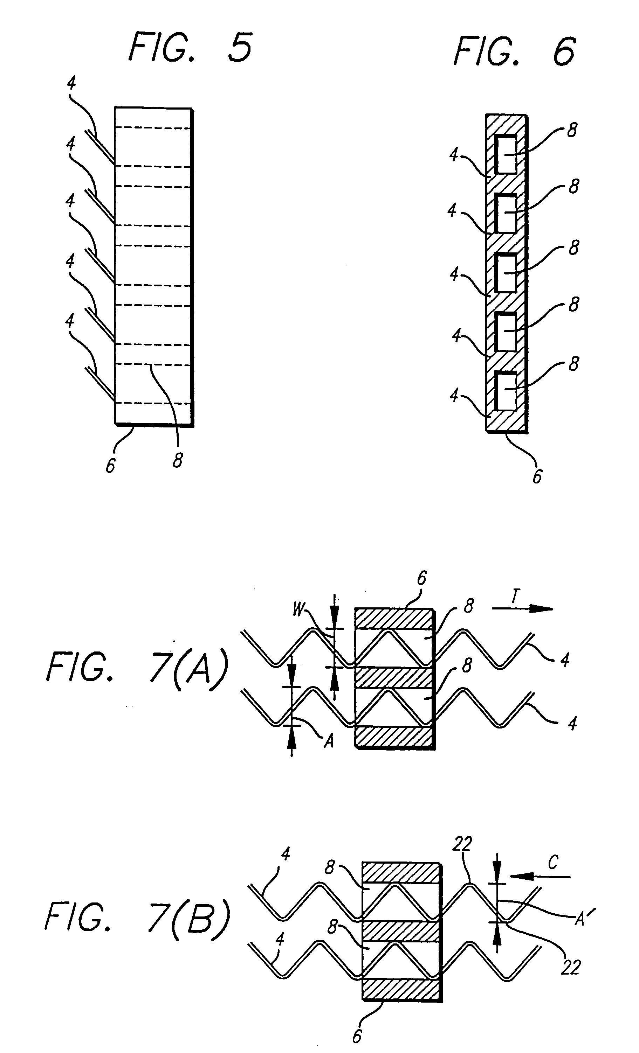

FIG. 4 is a partial plan view of an alternative embodiment showing enlarged ends 18 on the zig-zag struts 20. The enlarged ends 18 help to prevent the zig-zag struts 20 from traumatizing the vessel wall. The enlarged ends 18 also are conveniently pinched between the fingers of a technician, for pulling the zig-zag struts 20 through the slots 8.

Once the stent 2 is formed by curling the zig-zag struts 4 into the base section 6 to form the cylindrical hoop 12 as illustrated in FIG. 3, the stent is ready for use. First, the stent 2 must be loaded onto a delivery catheter or similar device. Specifically, a stent 2 embodying the present invention is placed over a stent delivery balloon catheter (not shown).

Second, the catheter is introduced percutaneously into a vessel, over a previously positioned guidewire in an over-the-wire angioplasty catheter system, and is tracked by a fluoroscope, until the balloon portion and the associated stent 2 are positioned at the point where the stent 2 is to be deployed. Third, the balloon portion (not shown), which is positioned directly beneath the stent 2, is inflated and the stent 2 is expanded by the balloon portion from a reduced diameter form to an expanded diameter form. Fourth, after the stent 2 is expanded to its final deployment expanded diameter, the underlying balloon is deflated and the catheter is withdrawn leaving the stent 2 in place.

As will be appreciated by those skilled in the art, the stent 2 while being transported is of a sufficiently small reduced diameter so as to allow it to be readily transported through a vessel. In an alternative embodiment, a retractable sheath (not shown) can cover the stent 2 to protect it during delivery to the deployment site where upon delivery the sheath then is retracted. Such protective sheaths for stents are known in the art.

FIGS. 5-7 provide more detailed views of the locking mechanism used in a preferred embodiment of the present invention. The locking mechanism, having a base section 6, is an important feature when the stent 2 is loaded onto the balloon catheter and the diameter of the stent 2 is expanded during deployment thereof and, when after implantation, the stent 2 encounters external forces that might cause the collapse of a more conventional locked stent.

FIG. 5 is a plan view of the preferred embodiment of the base section 6 showing the zig-zag struts 4 extending therefrom and a corresponding number of slots 8 therethrough. FIG. 6 is an end view illustrating the opposite end to that shown in FIG. 2. Ideally, the zig-zag struts 4 as seen in FIG. 6 are aligned in a coplanar manner as are the slots 8, although the two planes preferably are offset slightly from each other. The offset planes allow the zig-zag struts 4 to be curled around and to pass through the slots 8 corresponding to each.

FIGS. 7(a) and (b) illustrate the preferred embodiment of the locking mechanism of a stent according to the present invention in operation. In particular, FIG. 7(a) shows two zig-zag struts 4 under circumferential tension as indicated by the arrow T. The zig-zag struts 4 under this tensile force are stretched and pulled through the slots 8 within the base section 6.

In particular, under tension, the zig-zag struts 4 assume a small amplitude A, which is smaller than the width W of the slots 8. This change is due to the resilience in the zig-zag strut material, which enables the bends 22 to flatten out slightly under tension.

FIG. 7(a) depicts the process of loading a stent embodying the present invention onto a balloon catheter wherein the ends 10 are passed through the respective slots 8 and pulled to a diameter that is sufficiently small to fit over the outside diameter of the catheter balloon. Likewise, when the stent 2 is deployed, the increasing diameter of the underlying catheter balloon places the zig-zag struts 4 under tension, causing the struts to assume a small amplitude A. The small amplitude permits the individual bends 22 to pass unobstructed through the respective slots 8. In sum, the diameter of the hoop 12 can decrease if the zig-zag struts 4 are pulled on the ends 10, or the diameter can increase with the increasing diameter of the catheter balloon undergoing inflation.

After implantation, the catheter balloon is deflated and withdrawn through processes known in the art. Without the outwardly expanding catheter balloon to circumferentially tension the stent 2, the stent returns to the original, unstressed state and assumes the large amplitude A'. Because the large amplitude A' is greater than the slot width W, the bends 22 are obstructed from passing through. Hence, the diameter of the hoop 12 rigidly is locked in place and cannot be changed.

Once the stent 2 has been implanted, the stent may encounter external forces from the inner walls of the lumen or from some physical trauma originating from outside the patient. Such external forces tend to compress the zig-zag struts 4 circumferentially. This is illustrated by the arrow C in FIG. 7(b).

As mentioned above, in the unstressed state, the zig-zag struts 4 assume the larger amplitude A', which cannot pass through the slots 8. Under the compression force C, the zig-zag struts might be compressed slightly and the bends 22 might assume an even sharper angle and a corresponding large amplitude A' or greater. With the large amplitude A' or greater, which is greater than width W of the slot 8, the bends 22 as illustrated in FIG. 7(b) are obstructed and again cannot pass through the respective slots 8 in the base section 6. In fact, the bends 22 on either side of the base section 6 are locked in place. Consequently, the diameter of the hoop 12 is held constant despite encountering the external force. This enables the stent 2 embodying the invention to be crush-resistant.

The stent 2 preferably is made from a crush-resistant, shape-memory material such as a nickel-titanium alloy (NiTi). Compounds using NiTi are manufactured under the tradenames NITINOL and ELASTINITE and are available from several sources. The stent 2 could be made easier to use by the clinician by stabilizing the shape of the NiTi alloy in the state at which it is wound into a hoop 12 in order to take advantage of the shape-memory capacity of the material. The properties of shape-memory (pseudoelastic) NiTi are described in more detail in U.S. Patent Nos. 4,665,906; 5,067,957; and 5,190,546 to J. Jervis.

In an alternative embodiment, the longitudinal flexibility of the stent can be enhanced by constructing the stent from segments of three to five zig-zag struts and specifically joining these small segments together with a small connection of metal. In short, each zig-zag strut need not be a continuous piece of material but can be made from smaller individual segments.

According to one embodiment the stent can be made from standard stent materials such as stainless steel, tantalum, etc. In another embodiment the stent may be made from a biodegradable or bioabsorbable material such as polymers of the linear aliphatic polyester and glycolide families. Other materials contemplated for the stent include biocompatible polymers, such as of the type from the polyethylene, polyester, and polypropylene families and plastics such as a polymer from the linear aliphatic polyester family, such as poly(lactic acid), poly(glycolic acid), or polycaprolactone, and the associated copolymers, degradable polymers such as polyorthoester, polyanhydride, polydioxanone, and polyhydroxybutyrate.

## Claims

1. An intraluminal, rolled-up crush-resistant stent (2) for implantation in a body lumen, characterised by canprising:
a plurality of resilient, substantially parallel struts (4) having a zig-zag configuration and extending from a base section (6), wherein each zig-zag strut defines a large amplitude when the strut is in an unstretched state and a small amplitude (A) when the strut is in a stretched state;
a plurality of openings (8) formed within the base section corresponding to each of the zig-zag struts, each opening having a width that is less than the large amplitude; and
wherein the plurality of struts are curved and, when stretched to the small amplitude, pass through the openings of the base section to form a cylindrical hoop (12).

2. The crush-resistant stent of claim 1, wherein each zig-zag strut includes a thin rectangular cross-section.

3. The crush-resistant stent of claim 1, wherein each zig-zag strut includes a cylindrical cross-section.

4. The crush-resistant stent of claim 1, wherein the plurality of zig-zag struts have a flat cross-section.

5. The crush-resistant stent of claim 1, wherein each zig-zag strut includes a nickel-titanium alloy.

6. The crush-resistant stent of claim 1, wherein each strut includes an end opposite to the base section, the end having a disk shape.

7. The crush-resistant stent of claim 1, wherein the stent is made from a shape-memory alloy.

8. The crush-resistant stent of claim 1, wherein the stent includes a loop-shaped retainer binding an overlapping portion of the zig-zag struts.

9. The crush-resistant stent of claim 1, wherein the plurality of zig-zag struts are coplanar prior to being curved into a hoop.

10. An intraluminal, rolled-up crush-resistant stent for implantation in a body lumen, characterised by comprising:
at least one resilient, zig-zag shaped strut (4) curved to form a hoop (12), wherein each zig-zag has an amplitude that varies as the strut is stretched and relaxed;
a base section (6) disposed on the hoop having an opening for receiving the strut therethrough to close the hoop, whereby the curved strut passes through the base section and stretching the strut minimizes the amplitude to pass through the opening, which stretching defines a diameter of the hoop.

11. The crush-resistant stent of claim 10, wherein the amplitude of the stretched strut is smaller than a width of the opening.

12. The crush-resistant stent of claim 10, wherein the stretched strut defines a small amplitude and an unstretched strut defines a large amplitude, and wherein the small amplitude is less than the large amplitude.

13. The crush-resistant stent of claim 10, wherein the stent includes a plurality of coplanar, parallel zig-zag shaped struts.

14. The crush-resistant stent of claim 13, wherein the opening in the base section includes a plurality of parallel slots to receive the struts therethrough.

15. A process for loading a rolled-up crush-resistant stent onto a balloon portion of a catheter, comprising the method steps of:
providing a plurality of resilient, substantially parallel zig-zag struts (4) extending from a base section (6) and curved to form a hoop (12), wherein each zig-zag strut defines a large amplitude when the strut is in an unstretched state and a small amplitude when the strut is in a stretched state;
providing a plurality of openings within the base section corresponding to each of the zig-zag struts, each opening having a width that is less than the large amplitude;
stretching the struts to obtain the small amplitude;
passing the struts through the corresponding openings to form a closed hoop;
inserting the balloon portion into the hoop; and
releasing the struts so that the struts return to the unstretched state and assume the large amplitude.

16. The process of claim 15, wherein the process further comprises the step of further stretching the struts to further close the hoop upon the balloon portion.

17. The process of claim 15, wherein the process further comprises the step of curving the plurality of zig-zag struts so that each is coplanar with every other.

18. The process of claim 15, wherein each zig-zag strut has a flat cross-section.

19. The process of claim 15, wherein the process further comprises the step of inflating the balloon.

## Patentansprüche

1. Intraluminaler, aufgerollter, druckbeständiger Stent (2) zum Implantieren in ein Körperlumen, gekennzeichnet durch:
eine Mehrzahl elastischer, im Wesentlichen paralleler Streben (4), die eine Zickzack-Konfiguration haben und von einem Basisabschnitt (6) abstehen, wobei jede Zickzack-Strebe eine große Amplitude definiert, wenn die Strebe in einem ungestreckten Zustand ist, und eine kleine Amplitude (A), wenn die Strebe in einem gestreckten Zustand ist;
eine Mehrzahl von Öffnungen (8), die in dem Basisabschnitt entsprechend jeder der Zickzack-Streben ausgebildet ist, wobei jede Öffnung eine Breite hat, die kleiner als die große Amplitude ist; und
wobei die Mehrzahl der Streben gekrümmt ist und, wenn auf die kleine Amplitude gestreckt, durch die Öffnungen des Basisabschnitts unter Bildung eines zylindrischen Rings (12) hindurchgeht.

2. Druckbeständiger Stent nach Anspruch 1, wobei jede Zickzack-Strebe einen dünnen, rechteckigen Querschnitt aufweist.

3. Druckbeständiger Stent nach Anspruch 1, wobei jede Zickzack-Strebe einen zylindrischen Querschnitt aufweist.

4. Druckbeständiger Stent nach Anspruch 1, wobei die Mehrzahl der Zickzack-Streben einen flachen Querschnitt hat.

5. Druckbeständiger Stent nach Anspruch 1, wobei jede Zickzack-Strebe eine Nickel-Titan-Legierung enthält.

6. Druckbeständiger Stent nach Anspruch 1, wobei jede Strebe ein dem Basisabschnitt entgegengesetztes Ende aufweist, wobei das Ende scheibenförmig ist.

7. Druckbeständiger Stent nach Anspruch 1, wobei der Stent aus einer Formgedächtnis-Legierung gefertigt ist.

8. Druckbeständiger Stent nach Anspruch 1, wobei der Stent einen schleifenförmigen Halter aufweist, der einen überlappenden Abschnitt der Zickzack-Streben verbindet.

9. Druckbeständiger Stent nach Anspruch 1, wobei die Mehrzahl der Zickzack-Streben vor der Krümmung zu einem Ring koplanar ist.

10. Intraluminaler, aufgerollter, druckbeständiger Stent zum Implantieren in ein Körperlumen, gekennzeichnet durch:
zumindest eine elastische, zickzackförmige Strebe (4), die zur Bildung eines Rings (12) gekrümmt ist, wobei jeder Zickzack eine Amplitude hat, die sich verändert, wenn die Strebe gestreckt und entspannt wird;
einen an dem Ring angeordneten Basisabschnitt (6) mit einer Öffnung zur durchgehenden Aufnahme der Strebe, um den Ring zu schließen, wodurch die gekrümmte Strebe durch den Basisabschnitt hindurchgeht und ein Strecken der Strebe die Amplitude minimiert, um durch die Öffnung hindurchzugehen, und wobei das Strecken einen Durchmesser des Rings definiert.

11. Druckbeständiger Stent nach Anspruch 10, wobei die Amplitude des gestreckten Stents kleiner ist als eine Breite der Öffnung.

12. Druckbeständiger Stent nach Anspruch 10, wobei der gestreckte Stent eine kleine Amplitude definiert und der ungestreckte Stent eine große Amplitude definiert, und wobei die kleine Amplitude kleiner als die große Amplitude ist.

13. Druckbeständiger Stent nach Anspruch 10, wobei der Stent eine Mehrzahl koplanarer, paralleler, zickzackförmiger Streben aufweist.

14. Druckbeständiger Stent nach Anspruch 13, wobei die Öffnung in dem Basisabschnitt eine Mehrzahl paralleler Schlitze umfasst, um die hindurchgehenden Streben aufzunehmen.

15. Verfahren zum Aufsetzen eines aufgerollten, druckbeständigen Stents auf einen Ballonabschnitt eines Katheters, wobei das Verfahren die Schritte aufweist:
Vorsehen einer Mehrzahl elastischer, im Wesentlichen paralleler Zickzack-Streben (4), die von einem Basisabschnitt (6) abstehen und zur Bildung eines Rings (12) gekrümmt sind, wobei jede Zickzack-Strebe eine große Amplitude definiert, wenn die Strebe in einem ungestreckten Zustand ist, und eine kleine Amplitude, wenn die Strebe in einem gestreckten Zustand ist;
Vorsehen einer Mehrzahl von Öffnungen in dem Basisabschnitt entsprechend jeder der Zickzack-Streben, wobei jede Öffnung eine Breite hat, die kleiner als die große Amplitude ist;
Strecken der Streben zum Erhalt der kleinen Amplitude;
Durchführen der Streben durch die entsprechenden Schlitze zur Bildung eines geschlossenen Rings;
Einsetzen des Ballonabschnitts in den Ring; und
Lösen der Streben derart, dass die Streben in den ungestreckten Zustand zurückkehren und die große Amplitude einnehmen.

16. Verfahren nach Anspruch 15, wobei das Verfahren ferner den Schritt aufweist, die Streben weiter zu strecken, um den Ring auf dem Ballonabschnitt weiter zu schließen.

17. Verfahren nach Anspruch 15, wobei das Verfahren ferner den Schritt aufweist, die Mehrzahl von Zickzack-Streben zu krümmen, so dass jede zu jeder anderen koplanar ist.

18. Verfahren nach Anspruch 15, wobei jede Zickzack-Strebe einen flachen Querschnitt hat.

19. Verfahren nach Anspruch 15, wobei das Verfahren ferner den Schritt umfasst, den Ballon aufzupumpen.

## Revendications

1. Stent endoluminal enroulé (2), résistant à l'écrasement, destiné à être implanté dans une lumière corporelle, caractérisé en ce qu'il comprend :
une pluralité de montants élastiques (4), essentiellement parallèles, ayant une configuration en zig-zag et s'étendant à partir d'un tronçon de base (6), chaque montant en zig-zag définissant une grande amplitude lorsque le montant est dans un état non étiré et une petite amplitude (A) lorsque le montant est dans un état étiré ;
une pluralité d'ouvertures (8) formées dans le tronçon de base, correspondant à chacun des montants en zig-zag, chaque ouverture ayant une largeur moins grande que la grande amplitude ; et
les montants étant courbés et, lorsqu'ils sont étirés jusqu'à la petite amplitude, passant au travers des ouvertures du tronçon de base, pour former une boucle cylindrique (12).

2. Stent résistant à l'écrasement selon la revendication 1, dans lequel chaque montant en zig-zag a une section transversale rectangulaire mince.

3. Stent résistant à l'écrasement selon la revendication 1, dans lequel chaque montant en zig-zag comprend une section transversale cylindrique.

4. Stent résistant à l'écrasement selon la revendication 1, dans lequel les montants en zig-zag ont une section transversale plate.

5. Stent résistant à l'écrasement selon la revendication 1, dans lequel chaque montant en zig-zag comprend un alliage nickel-titane.

6. Stent résistant à l'écrasement selon la revendication 1, dans lequel chaque montant comprend une extrémité en regard du tronçon de base, l'extrémité ayant la forme d'un disque.

7. Stent résistant à l'écrasement selon la revendication 1, dans lequel le stent est fabriqué à partir d'un alliage à mémoire de forme.

8. Stent résistant à l'écrasement selon la revendication 1, dans lequel le stent comprend un élément de retenue en forme de boucle, reliant une partie en chevauchement des montants en zig-zag.

9. Stent résistant à l'écrasement selon la revendication 1, dans lequel les montants en zig-zag sont coplanaires les uns aux autres avant d'être courbés en une boucle.

10. Stent endoluminal enroulé, résistant à l'écrasement, destiné à être implanté dans une lumière corporelle, caractérisé en ce qu'il comprend :
au moins un montant élastique (4), en zig-zag, courbé pour former une boucle (12), chaque zig-zag ayant une amplitude qui varie lorsque le montant est étiré et relâché ;
un tronçon de base (6) disposée sur la boucle ayant une ouverture destinée à recevoir le montant à travers elle pour fermer la boucle, ce en conséquence de quoi le montant courbé traverse le tronçon de base, l'étirement du montant minimisant l'amplitude pour qu'elle passe par l'ouverture, lequel étirement définit un diamètre de la boucle.

11. Stent résistant à l'écrasement selon la revendication 10, dans lequel l'amplitude du montant étiré est plus petite qu'une largeur de l'ouverture.

12. Stent résistant à l'écrasement selon la revendication 10, dans lequel le montant étiré définit une petite amplitude et un montant non étiré définit une grande amplitude, et dans lequel la petite amplitude est moins grande que la grande amplitude.

13. Stent résistant à l'écrasement selon la revendication 10, dans lequel le stent comprend une pluralité de montant coplanaires, parallèles, en zig-zag.

14. Stent résistant à l'écrasement selon la revendication 13, dans lequel l'ouverture aménagée dans le tronçon de base comprend une pluralité de fentes parallèles destinées à recevoir les montants à travers elle.

15. Procédé de chargement d'un stent enroulé, résistant à l'écrasement, sur la partie ballonnet d'un cathéter, comprenant les étapes qui consistent à :
réaliser une pluralité de montants élastiques en zig-zag (4), essentiellement parallèles, s'étendant à partir d'un tronçon de base (6) et étant courbés de façon à former une boucle (12), chaque montant en zig-zag définissant une grande amplitude lorsque le montant est dans un état non étiré et une petite amplitude lorsque le montant est dans un état étiré ;
réaliser dans le tronçon de base une pluralité d'ouvertures correspondant à chacun des montants en zig-zag, chaque ouverture ayant une largeur moins grande que la grande amplitude ;
étirer les montants pour obtenir la petite amplitude ;
faire passer les montants au travers des ouvertures correspondantes pour former une boucle fermée ;
insérer la partie ballonnet dans la boucle ; et
relâcher les montants de telle sorte que ces derniers reviennent à leur état non étiré et prennent la grande amplitude.

16. Procédé selon la revendication 15, dans lequel le procédé comprend en outre l'étape consistant à étirer davantage les montants pour fermer davantage la boucle sur la partie ballonnet.

17. Procédé selon la revendication 15, dans lequel le procédé comprend en outre l'étape consistant à courber la pluralité de montants en zig-zag de telle sorte que les montants soient coplanaires les uns aux autres.

18. Procédé selon la revendication 15, dans lequel chaque montant en zig-zag a une section transversale plate.

19. Procédé selon la revendication 15, dans lequel le procédé comprend en outre l'étape consistant à gonfler le ballonnet.
